Europäisches Patentamt

European Patent Office

Office européen des brevets

(19)

(11) Publication number: **0 268 475 B1**

(12) # EUROPEAN PATENT SPECIFICATION

(45) Date of publication of patent specification: **19.11.92**

(51) Int. Cl.5: **C10G 32/02**, C10L 1/00, C12N 13/00

(21) Application number: **87310194.3**

(22) Date of filing: **19.11.87**

(54) **Magnetic method and apparatus.**

(30) Priority: **19.11.86 NZ 218331**

(43) Date of publication of application:
**25.05.88 Bulletin 88/21**

(45) Publication of the grant of the patent:
**19.11.92 Bulletin 92/47**

(84) Designated Contracting States:
**AT BE CH DE ES FR GB GR IT LI LU NL SE**

(56) References cited:
**US-A- 4 065 386**

**PATENT ABSTRACTS OF JAPAN, unexamined applications, section C, vol. 6, no. 102, June 11, 1982**

**THE PATENT OFFICE JAPANESE GOVERNMENT, page 113 C 107**

(73) Proprietor: **FORREST SCIENTIFIC RESEARCH LIMITED**
**112 Nelson Street**
**Petone, Wellington(NZ)**

(72) Inventor: **Forrest, Lindsay Warren**
**10 Benzie Avenue**
**Upper Hutt(NZ)**
Inventor: **Johnston, John Anthony**
**29 Parnell Street**
**Lower Hutt(NZ)**
Inventor: **Wickham, Colin Patrick**
**48B East Coast Road**
**Milford Auckland(NZ)**

(74) Representative: **Ranson, Arthur Terence et al**
**W.P. Thompson & Co. Coopers Building**
**Church Street**
**Liverpool L1 3AB(GB)**

EP 0 268 475 B1

# Description

This invention relates to a method and an apparatus for the control of biological growth and by-products thereof in hydrocarbon distillates. More particularly it relates to the control of microbiological growth and the production of organic acids in liquid-fuel systems, particularly systems where the fuel is gasoline, including aviation gasoline, or diesel fuels.

There are species of Protista which grow, and under certain circumstances even flourish within fuels and lubricants derived from the hydrocarbon distillate process. These use the above-mentioned fuels and lubricants as their source of nutrition.

"Protista" as used herein includes all lower organisms, such as algae, bacteria, fungi, and protozoans. The growth of such Protista within feed lines of such fuel systems, or in storage portions of such fuel systems causes impairment in systems. Typical features are blocked feed lines, clogged fuel filters, and accelerated wear of, for example, injection equipment due to secreted organic acids.

The use of liquid-fuelled internal combustion engines is widespread. For example, much transport of goods and commercial transport relies on such engines. The use of liquid-fuelled internal combustion engines in relation to such machines as hydraulic hoists, pumps, lifts and the like, and also in motors in heating and cooling systems is also common. While the major problems of Protistal growth currently relate principally to engines using gas, oil (diesel) and kerosine (jet fuel), this invention is equally applicable to any unit using fuels/lubricants derived from the crude oil distillation process.

A typical example of problems involving cooling systems is provided by refrigerated containers. These are commonly supplied with a small cooling system utilising a small internal combustion engine. The quality of the products transported in such containers, particularly food products, is dependent on the ability of the system to maintain a pre-selected temperature. The infestation of the fuel system with Protista decreases the efficiency of the system, occasionally to such a point that complete breakdown occurs.

Investigation into efficacious chemical additives for fuel systems to chemically control or eliminate the growth of these Protista continues, but the additives themselves may adversely affect the performance of the fuel, and do not deal with the accumulation of dead growth. In addition, it is a process entailing some considerable expense, and may require careful monitoring, or surveillance of chemical levels within the fuel itself. It is accordingly an object of this invention to provide an apparatus and a method for controlling Protistal growth and by-products thereof in hydrocarbon distillates, particularly in liquid fuels and lubricants.

The use of magnetic fields for treatment of fluids is known, principally in relation to the removal of scale forming or corrosive materials from fluids. For example, New Zealand Patents 172611, 94971, and 146614 deal with this aspect of magnetic treatment. In addition, the use of magnetic fields has been known in the treatment of fluids for removing ferromagnetic particles from fluids; New Zealand patent 156200 deals with this aspect of the use of magnetic fields.

One attempt to inhibit the growth of bacteria and algae in water using a magnetic field is disclosed in US patent 4065386. Here the water to be treated is passed through a device in which a magnetic field is generated transverse the direction of flow of the water. The device includes a stack of aligned, annular magnets arranged with the axis of the stack perpendicular to the direction of flow of the water. Also suggested is the use of a magnet surrounding a water duct. The device is intended for use in conjunction with swimming pool filters and conventional chemical treatment.

The applicant has discovered that magnetic fields are particularly effective in inhibiting growth of Protista in distillates. Further, the applicant has discovered that magnetic fields may be applied to the distillates either between pumping from storage to holding tanks, or between holding tanks and the combustion engine.

Accordingly, this invention provides a method of inhibiting protistal growth in a distillate characterised in that the distillate is passed through a plurality of magnets arranged in a stack in which each magnet is spaced a small distance from each adjacent magnet in the stack, the distillate being caused to flow between each pair of adjacent magnets in series in a direction transverse the flow direction so that the distillate passes in and out of a plurality of magnetic fields.

Preferably, the distillate is a hydrocarbon fuel.

The invention also provides an apparatus for carrying out the method. The apparatus comprises a housing having an inlet and an outlet; and magnetic means for producing a magnetic field, characterised in that the magnetic means comprises a plurality of magnets arranged in a stack in the housing, each magnet extending in a plane transverse the flow direction and being spaced a small distance from each adjacent magnet and that the apparatus has flow direction means to cause the distillate to flow between each pair of adjacent magnets in series transverse the flow direction.

A number of trials aboard commercial cargo ships and fishing vessels, where the problem is known to exist have been carried out and it has been discovered that, by passing the fuel of these vessels through the apparatus according to this invention, the rate of Protistal growth in the fuels of such vehicles is dramatically reduced.

It has been found that the length of magnetic field, its strength and the rate of flow of the distillates through the field are all factors relevant to the efficiency of the invention. Of prime importance is the entry and re-entry of the distillate into the, or several, magnetic fields. In order to achieve this object, a series of magnets are used in connection with the fluid flow, configured to achieve a multipass system which is characterised in that the liquid flows in and around an array of magnetic fields and baffles, thereby increasing the duration of the fluid flow contact with the magnets field. Thus, by an apparatus of relatively simple construction the efficiency of the magnetic field is enhanced.

It has been found that efficiency improves with increased time in the magnetic field. Desirably therefore, the apparatus is constructed to permit high volumes of flow, at a slow rate.

A variety of embodiments of the invention will now be described, by way of example only, with reference to the accompanying drawings, in which:

Fig.1 shows a side view of a partially cut away embodiment of the apparatus of the invention;

Fig.2 shows a base plate and a partially exploded stacking member of the apparatus of Fig.1;

Fig.3 shows a section through the centre of the same apparatus;

Fig.4 shows a section through a second embodiment;

Fig.5 shows a vertical section through a third embodiment;

Fig.6 shows a horizontal section through that apparatus, and

Fig.7 shows a plan view of the same embodiment.

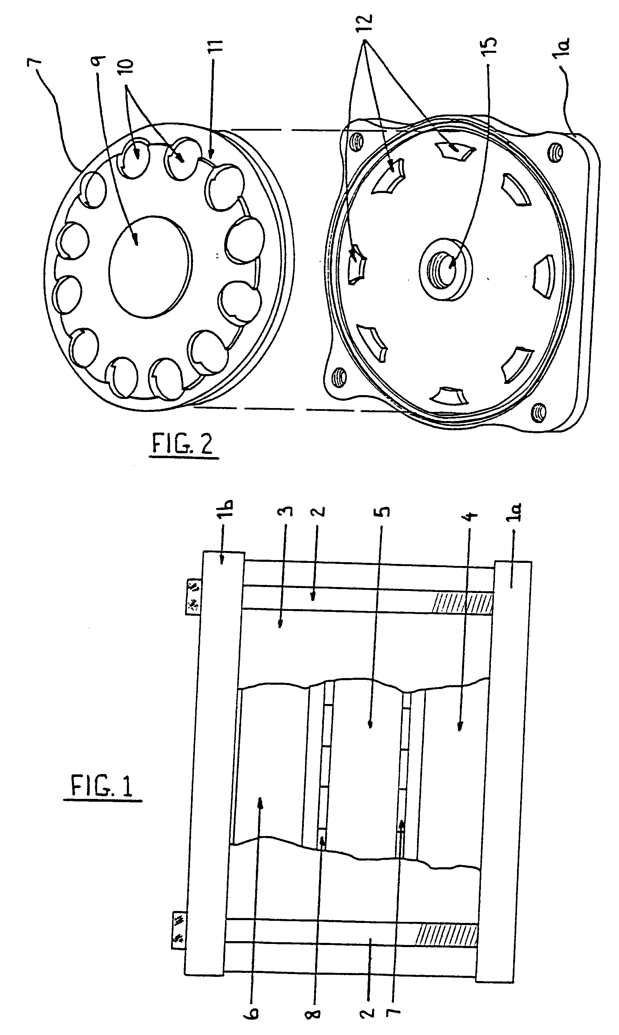

Fig.1 discloses a pair of mounting plates 1a and 1b, locked together by threaded restraining means 2. Side walls 3 are partially cut away to reveal within three substantially disc-shaped magnets stacked vertically, 4, 5 and 6. The magnets are spaced apart from each other by spacers 7 and 8. Such an arrangement is referred to herein as a "stack".

A perspective drawing of one of these spacers is shown in Fig.2. It has a central solid raised disc portion 9, and a series of spaced apart holes 10, close to the perimeter, and passing through a stepped, or shoulder portion 11.

Turning to mounting plate 1a shown in Fig. 2, it should be noted that there are a series of lands or spacers 12, upon which the lower magnet 4 sits.

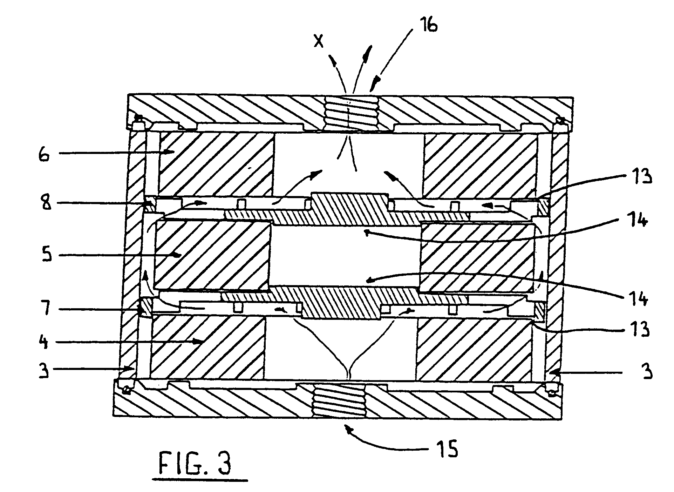

Fig.3 shows a section through the assemblage. It should be noted that the magnets, 4, 5 and 6 have a central hollow portion, which is coaxial with the centre of the spacers 7 and 8.

Said spacing members are substantially the same diameter as the diameter of the chamber formed by the side walls 3.

The outer periphery of each spacer 7, 8 contains a second stepped portion 13, which abuts and restrains magnets 6 and 4.

The spacers have a second raised disc portion 14, obverse from raised disc portion 9.

Raised portions 14 of spacers 7 and 8 are arranged facing each other, and dimensioned so as to fit tightly into the axial hole in magnet 5, thereby restraining it in position.

By virtue of the arrangements of the holes 10 in the spacers, the axial holes in the magnets 4, 5 and 6, and the spacing effect of spacers 7 and 8, a fuel may be passed through the apparatus, ingressing at aperture 15, through the axial hole of magnet 4, beneath spacer 7 and passing through holes 10 in spacer 7, around the cavity defined by the side walls 3 and the outer wall of magnet 5, through holes 10 in spacer 8, then into the axial hole of magnet 6, and egressing through aperture 16, and shown in Fig. 3 by direction arrows X.

In use, it is envisaged that apertures 15 and 16 will be threaded, to permit engagement with a threaded fuel line.

It will be seen that the fuel flow is therefore through the centre of magnets 4 and 6, and around the outer perimeter of magnet 5.

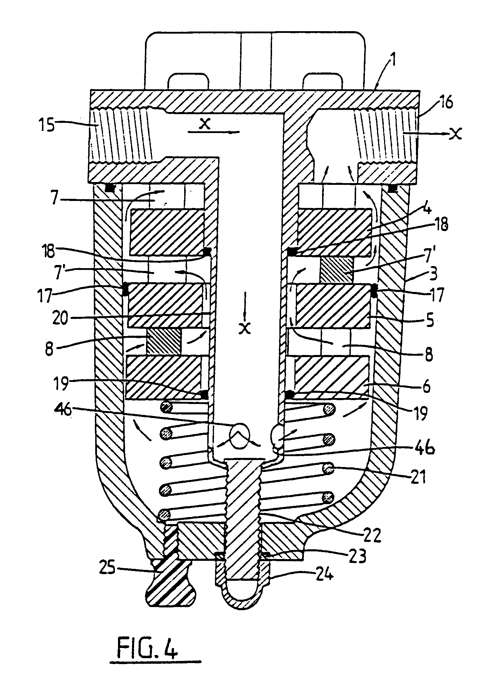

Fig.4 discloses a second embodiment, having a base plate 1, side walls 3, stacked magnets 4, 5 and 6, separated by sets of spacers 7, 7' and 8.

Each set of spacers is made up of three lugs around which the fuel may flow.

The magnets are arranged about an axial shaft 20 which has a threaded aperture 15 opening to a bore which itself opens by means of a plurality of egress holes 46.

A coil spring 21 is provided which retains the stack of magnets in place on shaft 20. Shaft 20 terminates in a threaded portion 22, which passes through walls 3 to be retained by means of a sealing washer and cap nut, 23 and 24 respectively.

A stop cock 25 is also provided for draining the apparatus. It will be seen that fuel may flow in entry aperture 15, through the bore, passing through the field created by each magnet, out through the holes 46, out across magnet 6 between side walls 3 and magnet 6, across magnet 5, between magnet 5 and the shaft 20, across magnet 4, between the walls 3 and magnet 4, and out through port 16. This flow direction is reversible

It will be appreciated that the flow thus winds in and out of the stack of magnets, moving in and out of the respective magnetic fields, and prolonging the duration of the period that the fluid is within a magnetic field.

Paired sealing means 17, by friction fit, retain magnet 5 in place: paired sealing means 18 and 19 similarly retain magnets 4 and 6 respectively, providing a fluid flow path X around the magnets. In the present embodiment, said sealing means are "O-rings".

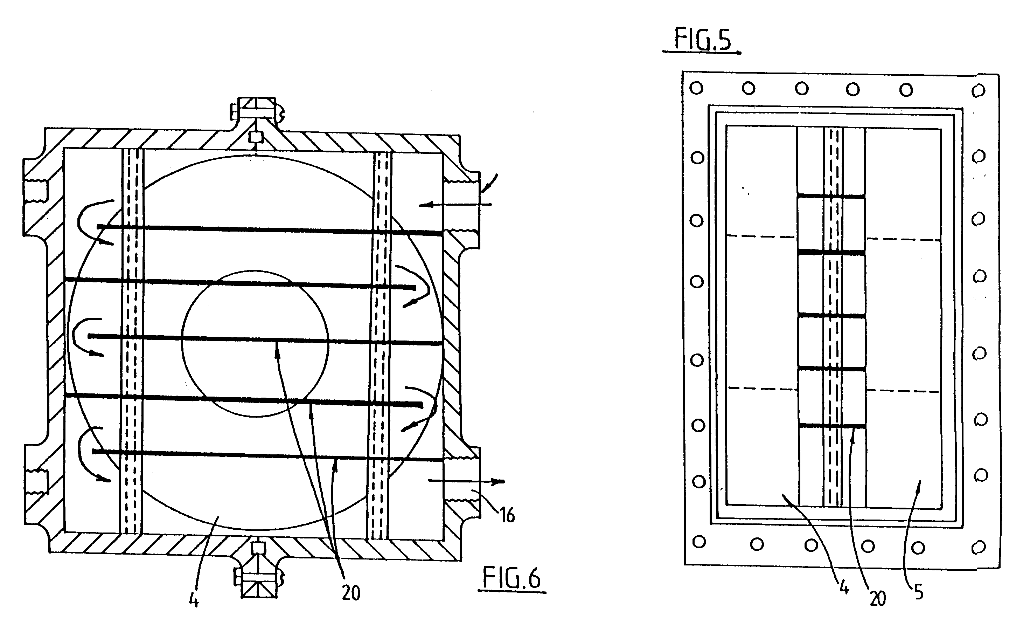

Fig.6 discloses an array of baffles 20 placed between a pair of magnets 4 and 5. The baffles are so arranged to permit fuel, entering in direction X of aperture 15 through the apparatus between the magnets and exiting at aperture 16.

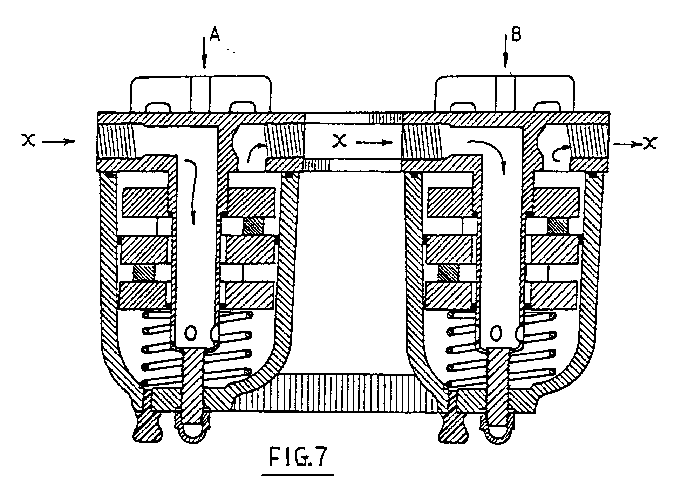

It will be appreciated that a multiplicity of units may be utilised. Fig.7 shows one embodiment of such a multistage system, in which, briefly, two units of constructions, similar to that depicted in Fig.6 are shown connected so that the flow egressing from a first unit A flows through the inlet of a second unit B, thus doubling the period of exposed field. The presently preferred embodiment, shown in Fig.7, has two such units, but the applicant contemplates a multistage apparatus having three or more of such units, connected serially.

## Claims

1. A method of inhibiting protistal growth in a distillate characterised in that the distillate is passed through a plurality of magnets arranged in a stack in which each magnet is spaced a small distance from each adjacent magnet in the stack, the distillate being caused to flow between each pair of adjacent magnets in series in a direction transverse the flow direction so that the distillate passes in and out of a plurality of magnetic fields.

2. A method according to claim 1 in which each alternate magnet (4, 5, 6) in the stack has a flow passage through it adjacent its centre, the distillate being caused to flow around the peripheral edge of the preceding magnet, through the flow passage, and around the peripheral edge of the next magnet.

3. An apparatus for carrying out the method according to claim 1 or claim 2, the apparatus comprising a housing having an inlet (15) and an outlet (16); and magnetic means for producing a magnetic field, characterised in that the magnetic means comprises a plurality of magnets (4, 5, 6) arranged in a stack in the housing, each magnet extending in a plane transverse the flow direction and being spaced a small distance from each adjacent magnet and that the apparatus has flow direction means (7, 7', 8, 17, 18, 19 and 28) to cause the distillate to flow between each pair of adjacent magnets (4, 5, 6) in series transverse the flow direction.

4. An apparatus according to claim 3 in which each alternate magnet (4, 5, 6) in the stack has a bore through its centre and the flow direction means (7, 7', 8, 17, 18, 19 and 28) are so arranged to cause the distillate to flow through the bore of a magnet and around the outer periphery of the next magnet.

5. An apparatus according to claim 4 in which each of the magnets (4, 5, 6) in the stack is mounted on a tubular shaft (20) and the flow direction means (7, 7', 8, 17, 18, 19 and 28) are so arranged to cause the distillate to flow around the outer periphery of each magnet and through the bore of each alternate magnet.

6. An apparatus according to claim 4 or claim 5 in which the flow direction means (7, 7', 18, 19 and 28) are so arranged to cause the distillate to flow across the face of one or more of the magnets (4, 5, 6) normal to the direction of flow through the apparatus.

7. An apparatus according to claim 5 or claim 6 in which the magnets (4, 5, 6) are held on the tubular shaft (20) by a spring (21) that is concentric with the tubular shaft and is biased between the wall (3) of the housing and the magnets (4, 5, 6).

## Patentansprüche

1. Verfahren zur Hemmung des Protisten-Wachstums in einem Destillat,
   **dadurch gekennzeichnet,**
   daß das Destillat durch mehrere in einem Stapel angeordnete Magnete fließt,
   wobei jeder Magnet in dem Stapel durch einen kleinen Abstand von denbenachbarten Magneten getrennt ist und wobei das Destillat so geführt wird, daß es der Reihe nach in einer Richtung transversal zur Flußrichtung zwischen jedem Paar benachbarter Magnete hindurchfließt, so daß das Destillat in mehrere Magnetfelder hinein und aus diesen heraus fließt.

2. Verfahren nach Anspruch 1,
   wobei jeder zweite Magnet (4,5,6) in dem Stapel eine Durchflußöffnung in der Nähe seines Zentrums aufweist und das Destillat so geführt wird, daß es um die periphere Kante des vorhergehenden Magnetes durch die Durchflußöffnung und um die periphere Kante des nächsten Magnetes fließt.

**3.** Vorrichtung zur Durchführung des Verfahrens nach Anspruch 1 oder 2,
wobei die Vorrichtung aufweist:
ein Gehäuse mit einer Einflußöffnung (15) und einer Ausflußöffnung (16) und eine Magnetvorrichtung zur Erzeugung eines Magnetfeldes, **dadurch gekennzeichnet,**
daß die Magnetvorrichtung mehrere Magnete (4,5,6) aufweist, die in einem Stapel in dem Gehäuse angeordnet sind, wobei sich jeder Magnet in einer Ebene transversal zur Flußrichtung erstreckt und durch kleine Abstände von jedem benachbarten Magneten getrennt ist, und
daß die Vorrichtung eine Flußführungseinrichtung (7,7',8,17,18,19 und 28) aufweist um zu bewirken, daß das Destillat nacheinander transversal zur Flußrichtung zwischen jedem Paar benachbarter Magnet (4,5,6) hindurchfließt.

**4.** Vorrichtung nach Anspruch 3,
wobei jeder zweite Magnet (4,5,6) in dem Stapel eine Bohrung durch sein Zentrum aufweist und die Flußführungseinrichtung (7,7',8,17,18,19 und 28) so angeordnet sind, daß das Destillat durch die Bohrung eines Magneten und um die äußere Peripherie des nächsten Magneten fließt.

**5.** Vorrichtung nach Anspruch 4,
wobei sämtliche Magnet (4,5,6) in dem Stapel an einem röhrenförmigen Schaft (20) befestigt sind und die Flußführungseinrichtung (7,7',8,17,18,19 und 28) so angeordnet ist, daß das Destillat um die äußere Pheripherie eines jeden Magneten und durch die Bohrungen jedes zweiten Magneten fließt.

**6.** Vorrichtung nach Anspruch 4 oder 5,
wobei die Flußführungseinrichtung (7,7',8,17,18,19 und 28) so angeordnet ist, daß das Destillat über die Stirnfläche eines oder mehrerer der Magnete (4,5,6) senkrecht zur Flußrichtung durch die Vorrichtung fließt.

**7.** Vorrichtung nach Anspruch 5 oder 6,
wobei die Magnete (4,5,6) andem röhrenförmigen Schaft (20) durch eine Feder (21) gehaltert werden, die konzentrisch zu dem röhrechenförmigen Schaft angeordnet ist und zwischen die Wandung (3) des Gehäuses und die Magnete (4,5,6) gespannt ist.

**Revendications**

**1.** Procédé d'inhibition de la croissance protistale dans un distillat, caractérisé en ce que l'on fait passer le distillant à travers une pluralité d'aimants disposés en un empilage dans lequel chaque aimant est espacé à une faible distance de l'aimant adjacent dans l'empilage, le distillat s'écoulant entre chaque couple d'aimants adjacents, en série, dans une direction transversale à la direction de l'écoulement, de sorte qu'il entre et sort d'une pluralité de champs magnétiques.

**2.** Procédé selon la revendication 1, dans lequel chaque aimant alterné (4,5,6) situé dans l'empilage présente un passage d'écoulement passant adjacent à son centre, le distillat s'écoulant autour du bord périphérique de l'aimant précédent, à travers le passage d'écoulement, et autour du bord périphérique de l'aimant qui suit.

**3.** Dispositif pour mettre en oeuvre le procédé selon la revendication 1 ou 2, la dispositif comprenant un carter présentant une entrée (15) et une sortie (16); et des moyens magnétiques pour produire un champ magnétique, caractérisé en ce que les moyens magnétiques comprennent une pluralité d'aimants (4,5,6) disposés en empilage dans le carter, chaque aimant s'étendant dans un plan transversal par rapport à la direction de l'écoulement et étant espacé d'une faible distance par rapport à chaque aimant adjacent et en ce que le dispositif présente des moyens d'orientation d'écoulement (7, 7', 8, 17, 18, 19 et 28) destinés à faire s'écouler le distillat entre chaque couple d'aimants adjacents (4,5,6) en série, transversalement par rapport à la direction de l'écoulement.

**4.** Dispositif selon la revendication 3, dans lequel chaque aimant alterné (4,5,6) dans l'empilage présente un trou traversant passant par son centre et les moyens d'orientation d'écoulement (7, 7', 8, 17, 18, 19 et 28) étant disposés de façon à faire s'écouler le distillat à travers le trou d'un aimant et autour de la périphérie extérieure de l'aimant qui suit.

**5.** Dispositif selon la revendication 4, dans lequel chacun des aimants (4,5,6) dans l'empilage est monté sur une tige tubulaire (20) et les moyens d'orientation d'écoulement (7, 7', 8, 17, 18, 19 et 28) sont disposés de façon à faire s'écouler le distillat autour de la périphérie extérieure de chaque aimant et à travers le trou de chaque aimant alterné.

**6.** Dispositif selon la revendication 4 ou 5, dans lequel les moyens d'orientation d'écoulement (7, 7', 8, 17, 18, 19 et 28) sont disposés de façon à faire s'écouler le distillat sur la surface d'un ou plusieurs des aimants (4,5,6), perpendiculairement à la direction de l'écoulement dans le dispositif.

7. Dispositif selon la revendication 5 ou 6, dans lequel les aimants (4,5,6) sont maintenus sur la tige tubulaire (20) à l'aide d'un ressort (21) concentrique par rapport à la tige tubulaire et comprimé entre la paroi (3) du carter et les aimants (4,5,6).

FIG. 2

FIG. 1

FIG. 3

FIG. 4

FIG.5

FIG.6

EP 0 268 475 B1

FIG.7